Europäisches Patentamt

European Patent Office

Office européen des brevets

(11) Veröffentlichungsnummer: **0 060 491**
**B1**

(12) **EUROPÄISCHE PATENTSCHRIFT**

(45) Veröffentlichungstag der Patentschrift: **12.06.85**

(51) Int. Cl.⁴: **C 07 K 15/06**

(21) Anmeldenummer: **82101843.9**

(22) Anmeldetag: **09.03.82**

(54) Protein (PP₁₆), Verfahren zu seiner Anreicherung und Gewinnung sowie seine Verwendung

(30) Priorität: **13.03.81 DE 3109629**

(43) Veröffentlichungstag der Anmeldung:
**22.09.82 Patentblatt 82/38**

(45) Bekanntmachung des Hinweises auf die
Patenterteilung:
**12.06.85 Patentblatt 85/24**

(84) Benannte Vertragsstaaten:
**AT BE CH DE FR GB IT LI LU NL SE**

(56) Entgegenhaltungen:
**EP-A-0 000 134**
**EP-A-0 009 715**
**EP-A-0 014 756**
**DE-A-2 640 387**

(73) Patentinhaber: **BEHRINGWERKE**
**Aktiengesellschaft**
**Postfach 1140**
**D-3550 Marburg 1 (DE)**

(72) Erfinder: **Bohn, Hans, Dr.**
**Oberer Eichweg 26**
**D-3550 Marburg/Lahn (DE)**

(74) Vertreter: **Meyer-Dulheuer, Karl-Hermann,**
**Dr. et al**
**HOECHST Aktiengesellschaft Zentrale**
**Patentabteilung Postfach 80 03 20**
**D-6230 Frankfurt/Main 80 (DE)**

Courier Press, Leamington Spa, England.

# Description

Die Erfindung betrifft ein neues Protein ($PP_{16}$) Verfahren zu seiner Anreicherung und Gewinnung sowie seine Verwendung.

In Extrakten menschlicher Organe und besonders Plazenten wurde bereits eine Reihe löslicher Proteine, die aus diesen Geweben stammen, nachgewiesen (Bohn, H., Placental and Pregnancy Proteins, in Carcino-Embryonic Proteins, Vol. 1, Ed. F. G. Lehmann, Elsevier/North-Holland Biomedical Press, 1979).

Die vorliegende Erfindung beschreibt die Isolierung und Charakterisierung eines neuen löslichen Proteins, genannt $PP_{16}$.

$PP_{16}$ kommt im Extrakt einiger menschlicher Organe wie Plazenta, Milz, Magen und Lunge vor. Aus einer ausgewachsenen menschlichen Plazenta (600 g) lassen sich mit physiologischer Salzlösung im Durchschnitt 22 mg dieses Proteins extrahieren. In ähnlicher Größenordnung dürfte die Konzentration dieses Proteins in Milz, Magen und Lunge von erwachsenen Menschen sein. Extrakte anderer Organe enthalten dieses Protein nicht oder in wesentlich geringerer Konzentration. Auch im Serum und anderen Körperflüssigkeiten des Menschen kommt $PP_{16}$ normalerweise nicht oder nur in Spuren ($< 1$ mg/l) vor.

Gegenstand der Erfindung ist das Protein $PP_{16}$, gekennzeichnet durch

a) einen Kohlenhydratanteil von $4,3\pm1,6\%$ bestehend aus $3,4\pm1,2\%$ Hexosen, $0,24\pm0,07\%$ Hexosaminen, $0,06\pm0,03\%$ Fucose und $0,6\pm0,3\%$ Neuraminsäure;

b) einen Sedimentationskoeffizientien $S_{20,w}^{0}$ von $4,6\pm0,4$ S;

c) ein im Natriumdodecylsulfat (SDS)-haltigen Polyacrylamidgel bestimmtes Molekulargewicht von $46\,000\pm3\,000$.

d) einen Extinktionskoeffizienten $E_{1\,cm}^{1\%}$ (280 nm) von $8,82\pm0,5$;

e) eine elektrophoretische Beweglichkeit im Bereich des Albumins und

f) einen isoelektrischen Punkt von $4,7\pm0,2$.

Zur Erläuterung der kennzeichnenden Merkmale des Proteins sei folgendes ausgeführt:

Der Sedimentationskoeffizient wurde in einer analytischen Ultrazentrifuge der Firma Beckman (Spinco-Apparatur, Modell E) bei 60.000 UpM in Doppel sektorzellen mit Hilfe der UV-Scanner Technik bei 280 nm bestimmt. Als Lösungsmittel diente ein 0,05 M Phosphatpuffer (pH 6,8), der 0,2 Mol/l NaCl enthielt. Die Proteinkonzentration wurde auf eine optische Dichte von etwa 3 eingestellt. Der Sedimentationskoeffizient wurde auf die Basis von Wasser bei 20°C umgerechnet.

Zur Bestimmung des Molekulargewichts im SDS—PAA—Gel wurde ein Gel mit 7,5% Polyacrylamid (PAA), das 0,1% Natriumdodecylsulfat (SDS) enthielt, verwendet. Als Vergleichssubstanz dienten humanes Plazentalactogen (HPL) und Human-Albumin sowie dessen Aggregate.

Zur Bestimmung des Extinktionskoeffizienten wurde die Substanz 0,10 % ig (w:v) in destilliertem Wasser gelöst.

Die elektrophoretische Beweglichkeit wurde in der Mikromodifikation mit dem Gerät Microzone® R 200 von Beckman Instruments auf Zelluloseacetatfolien (Firma Sartorius, Göttingen, Bundesrepublik Deutschland) unter Verwendung von Natriumdiäthylbarbiturat-Puffer (pH 8,6) bestimmt.

Die Bestimmung der Kohlenhydrate erfolgte nach der von H. E. Schultze, R. Schmidtberger, H. Haupt, Biochem. Z. *329*, Seite 490 (1958), beschriebenen Methode.

Die Aminosäurenanalyse wurde nach S. Moore, D. H. Spackman, W. H. Stein, Anal. Chem. *30*, Seite 1185 (1958), unter Verwendung des Flüssigkeitschromatographen Multichrom® B der Firma Beckman durchgeführt. Cystin wurde nach Oxydation des Proteins mit Perameisensäure (S. Moore et al., Anal. Chem., *238*, Seite 235 (1963)) als Cysteinsäure bestimmt. Der Tryptophangehalt ist direkt photometrisch nach H. Edelhoch, Biochemistry *6*, Seite 1948 (1967), ermittelt worden.

Tabelle I enthält des Ergebnis der Aminosäurenanalyse von $PP_{16}$

### Tabelle I
### Aminosäurenzusammensetzung von PP$_{16}$

| | Reste pro 100 Reste (Mol-%) | VK% *) |
|---|---|---|
| Lysin | 5,80 | 9,58 |
| Histidin | 2,66 | 9,26 |
| Arginin | 4,35 | 10,18 |
| Asparaginsäure | 12,64 | 4,27 |
| Threonin | 4,00 | 9,01 |
| Serin | 6,04 | 5,16 |
| Glutaminsäure | 12,54 | 2,42 |
| Prolin | 5,37 | 1,57 |
| Glycin | 5,76 | 6,12 |
| Alanin | 6,09 | 6,44 |
| Cystin/2 | 2,34 | 3,57 |
| Valin | 5,48 | 6,16 |
| Methionin | 2,25 | 12,44 |
| Isoleucin | 5,68 | 0,61 |
| Leucin | 7,78 | 3,89 |
| Tyrosin | 4,17 | 3,79 |
| Phenylalanin | 5,93 | 3,96 |
| Tryptophan | 1,05 | 11,42 |

*) VK=Variationskoeffizient

PP$_{16}$ hat folgende Eigenschaften, die sich in einem Verfahren zu seiner Isolierung verwenden lassen, indem diesen Eigenschaften entsprechende Maßnahmen getroffen werden:

1) Mit Ammoniumsulfat wird es bei pH 7,0 und 30—60% Sättigung aus wässrigen Lösungen gefällt.

2) Mit wasserlöslichen Acridinbasen, z.B. 2-Äthoxy-6,9-diaminoacridinlactat wird es bei pH-Werten zwischen 4—9 und einer Konzentration der Base von 0,2 bis 0,8% w/v präzipitiert.

3. Unter den Bedingungen einer Euglobulinfällung, nämlich durch Einstellen eines pH-Wertes von 5—6 in einer verdünnten Salzlösung bleibt es zur Hauptsache im Überstand.

4) Bei der elektrophoretischen Auftrennung zeigt PP$_{16}$ bei pH 8,0 eine ähnliche Beweglichkeit wie Albumin.

5. Bei der Gelfiltration mit Sephadex® verhält es sich wie Proteine mit Molekulargewichten von 20 000 bis 70 000.

6) Es läßt sich an schwach basische Ionenaustauscher wie beispielsweise DEAE-Cellulose oder DEAE-Sephadex® bei einer Leitfähigkeit von etwa 0—2 mS und einem pH-Wert von etwa pH 7 bis 9 binden und wird erste unter Verwendung stärker konzentrierter Salzlösungen (1—5% NaCl-Lösungen) vom Ionenaustauscher wieder eluiert.

Gegenstand der Erfindung ist ferner ein Verfahren zur Gewinnung des PP$_{16}$, dadurch gekennzeichnet, daß eine Lösung, die dieses Protein enthält, unter Verwendung der obenstehenden Eigenschaften fraktioniert wird.

Lösungen, die PP$_{16}$ enthalten, erhält man durch Extraktion von Organen, in denen dieses Protein vorkommt. Dafür sing reife menschliche Plazenten, wie sie bei der Geburt anfallen, vorzugsweise geeignet. Aber auch andere Organe wie Milz, Magen oder Lunge können dafür eingesetzt werden. Das Gewebe wird mechanisch zerkleinert und dann mit Wasser oder salzhaltigen Lösungen verrührt. Der Geweberückstand wird abzentrifugiert. Der Überstand enthält die löslichen Gewebeproteine.

Neben Ammoniumsulfat können selbstverständlich auch andere in der präparativen Biochemie üblicherweise eingesetzte Neutralsalze zur Ausfällung des PP$_{16}$ verwendet werden. Neben einer Acridinbase ist auch ein wasserlösliches Derivat einer Chinolinbase, wie sie für Proteinfraktionierungen bekannt sind, im Rahmen des erfingsgemäßen Verfahren einsetzbar. Außer seinem elektrophoretischen Verhalten oder seinem Molekulargewicht sind zur Isolierung des Proteins auch andere Maßnahmen brauchbar, die geeignet sind, ein Protein mit den angegebenen Eigenschaften von anderen Proteinen abzutrennen. Hierzu können die verschiedenen Methoden der Gelfiltration, Gelchromatographie oder Ultrafiltration oder auch die Eigenschaft des PP$_{16}$, aus verdünnten Pufferlösungen an schwach basische Ionenaustauscher gebunden und hiervon mit stärker konzentrierten Salzlösungen wieder eluiert werden zu können, verwendet werden.

Durch eine zweckmäßige Kombination der genannten Maßnahmen, die eine Anreicherung des PP$_{16}$ oder eine Abtrennung dieses Proteins von anderen Proteinen bewirken, kann PP$_{16}$ isoliert werden.

Das so durch kombinierte Methoden der Fraktionierung gewonnene Protein enthält in Spuren immer noch andere Proteine als Verunreinigungen. Diese können mit entsprechenden Immunadsorbentien, das heißt mit trägergebundenen Antikörpen gegen diese Begleitproteine, entfernt werden.

Demzufolge ist ein Gegenstand der vorliegenden Erfindung in den einzelnen Schritten zur Anreicherung des PP$_{16}$ und in dem durch Kombination der Maßnahmen zur Anreicherung sich ergebenden Verfahren zur Reinigung des PP$_{16}$ zu sehen.

Das Verfahren zur Anreicherung ist gekennzeichnet durch die Anwendung der den obengenannten Eigenschaften 1 bis 6 entsprechenden Maßnahmen deren chemischen oder biochemischen präparativen Äquivalente und einer Hochreinigung durch Verwendung von Immunadsorbentien.

Gegenstand der Erfindung ist weiterhin ein Verfahren zur Herstellung des PP$_{16}$, dadurch gekennzeichnet, daß man eine Flüssigkeit, die dieses Protein enthält, einer oder mehreren Verfahrensmaßnahmen, die zur Isolierung von Proteinen bekannt sind, unterwirft und jeweils das Material gewinnt, in welchem das Protein mit den Merkmalen des PP$_{16}$ vorliegt.

Zum Nachweis und zur Bestimmung des PP$_{16}$ etwa in einer Fraktion aus einer Trennoperation können immunchemische Methoden dienen, da PP$_{16}$ antigene Eigenschaften hat.

Zum immunologischen Nachweis von PP$_{16}$ kann die Geldiffusionstechnik nach Ouchterlony (siehe beispielsweise Schultze and Heremans, Molecular Biology of Human Proteins, Vol. 1, 134) herangezogen werden.

Ein für diesen Zweck brauchbares Antiserum kann folgendermaßen gewonnen werden: Durch Immunisieren von Kaninchen mit einer PP$_{16}$-enthaltenden Plazentaproteinfraktion (Mutterlaugen aus der Kristallisation von humanem Plazentalactogen (HPL) nach Bohn, H., Experientia 27 (1971), (1223) wird ein polyvalentes Antiserum erhalten, mit dem PP$_{16}$ nachgewiesen werden kann. Dieses Antiserum kann durch Absorption mit normalem menschlichen Serum und solchen Plazentafraktionen, die PP$_{16}$ nicht enthalten, oder Proteinen, beispielsweise mit HPL, gegen das Antigen PP$_{16}$ weitgehend spezifisch gemacht werden.

Mit Hilfe des nach der vorliegenden Anmeldung gewonnenen reinen PP$_{16}$ lassen sich durch Immunisieren von Tieren nach bekannten Methoden monospezifische Antiseren herstellen.

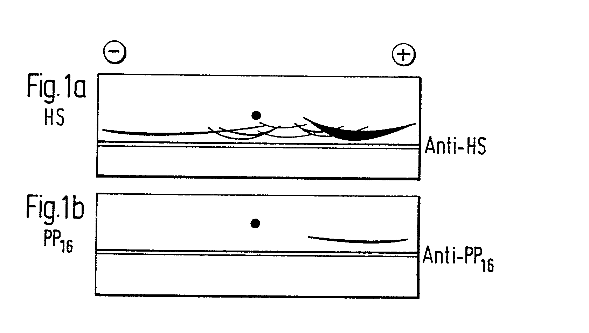

Abbildung 1 b) zeigt die immunologische Reaktion von PP$_{16}$ mit einem spezifischen Antiserum von Kaninchen nach Auftrennung im elektrischen Feld in Agar-haltigem Gel.

Abbildung 1 a) bringt zum Vergleich dazu die Auftrennung der Proteine des Serums, sichtbar gemacht durch deren Immunrektion mit einem Antiserum vom Kaninchen gegen Humanserum (HS).

Der Nachweis und die Bestimmung von PP$_{16}$ mit immunologischen Methoden hat diagnostische Bedeutung:

PP$_{16}$ ist ein Protein, das in einigen Organen des menschlichen Körpers (z.B. Plazenta, Milz, Magen und Lunge) in größerer Konzentration vorkommt. Im Blut läßt es sich normalerweise nicht, das heißt nur in Spuren (<1 mg/l) nachweisen. Bei Erkrankungen, die mit dem Zerfall von PP$_{16}$-haltigen Gewebszellen einhergehen, kann dieses Protein in erhöhter Konzentration im Serum oder in anderen Körperflüssigkeiten, beispielsweise Urin, erscheinen. Der Nachweis und die Bestimmung dieses Proteins kann dann zum diagnostischen Nachweis einer Erkrankung oder zur Überwachung des Krankheitsverlaufs und zur Kontrolle der Therapie einer solchen Erkrankung dienen.

PP$_{16}$ kann also verwendet werden, um Antiseren herzustellen, di dazu dienen können, PP$_{16}$ nachzwweisen und zu bestimmen.

Die Erfindung wird am nachstehenden Beispiel erläutert:

Beispiel

A) Extraktion der Plazenten und Fraktionierung des Extraktes mit Rivanol®, Hoechst AG, und Ammoniumsulfat

1.000 kg tiefgefrorene menschliche Plazenten werden im Schneidmischer zerkleinert und mit 1.000 l einer 0,4 %igen (w/w) Kochsalzlösung extrahiert. Der Extrakt wird, nach Abtrennung des Geweberückstandes durch Zentrifugation, mit 20 %iger (w/w) Essigsäure auf pH 6,0 eingestellt und unter Rühren mit 200 l einer 3 %igen (w:w) Lösung von 2-Äthoxy-6,8-Diaminoacridin-Lactat versetzt.

Den durch Zentrifugation abgetrennten Niederschlag versetzt man mit 500 l einer 2,5 %igen (w:w) NaCl-Lösung, rührt 4 Stunden und zentrifugiert von abgeschiedenen Chlorid des 2-Äthoxy-6,9-Diaminoacridins ab. Die Lösung versetzt man unter Rühren langsam mit soviel festem Ammoniumsulfat, daß eine Endkonzentration von 30% (w/v) erreicht wird, wodurch PP$_{16}$ zusammen mit anderen Proteinen ausfällt. Der Niederschlag wird abzentrifugiert. Man erhält etwa 4,5 kg einer feuchten Paste, die im Nachfolgenden als Fraktion A bezeichnet wird.

B) Gelfiltration an Sephadex G-150®

1.500 g der Fraktion A werden in Wasser gelöst und gegen einen 0,01 M Tris-HCl-Puffer (pH 8,0), der 0,05% NaN$_3$ enthält (Pufferlösung I), dialysiert. Die zurückbleibende Lösung wird auf eine mit Sephadex G-150® gefüllte Säule (60×56 cm) aufgetragen und mit Pufferlösung I eluiert. Die Eluate werden im Geldiffusionstest nach Ouchterlony mit einem spezifischen Anti-PP$_{16}$ Kaninchemserum geprüft. Die PP$_{16}$ enthaltenden Fraktionen werden gesammelt und als Fraktion B bezeichnet.

C) Chromatographie an DEAE-Cellulose

Fraktion B wird an DEAE-Cellulose (Säule 10×28 cm) adsorbiert. Man spült die Säule mit Pufferlösung I und eluiert mit 0,85 %iger (w:v) Kochsalzlösung solange, bis im Durchlauf mit Trichloressigsäure keine Fällung mehr entsteht. Anschließend wird die Säule mit 5% NaCl-Lösung eluiert. Aus diesem zweiten Eluat werden die Proteine durch Zusatz von soviel Ammoniumsulfat, daß die Konzentration 30% (w/v) beträgt, ausgefällt. Der Niederschlag wird abzentrifugiert (Fraktion C).

D) Euglobulinfällung

Fraktion C wird in Wasser gelöst und gegen Pufferlösung I dialysiert. Man stellt die Lösung durch Zugabe von 2 N Essigsäure unter Rühren auf pH 5,5 ein. Der Niederschlag, der im wesentlichen nur Begleitproteine enthält, wird abzentrifugiert. Die Proteine im Überstand werden durch Einengen auf einem Ultrafilter oder durch Fällen mit Ammoniumsulfat (30% w/v) ankonzentriert und gegen einen 0,1 M Ammoniumbikarbonatpuffer dialysiert (Fraktion D).

E) Präparative Zonenelektrophorese

Fraktion D wird in eine Apparatur zur präparativen Elektrophorese, wie sie beispielsweise von N. Heimburger und R. Schmidtberger in Behringwerke-Mitteilungen, Heft 43, Seite 83 ff., insbesondere auf Seite 119—120, beschrieben wird, eingetragen. Bei dem Gerät handelt es sich um die horizontale Anordnung einer Trägerelektrophorese in einem offenen Trog, in welchem das Trägermaterial zur Abführung der bei der Elektrophorese auftretenden Joule'schen Wärme auf unter 10°C gekühlt wird. Als Trägermaterial dienen gegenüber Proteinen indifferente Substanzen, vorteilhaft Polyvinylchlorid, oder dessen Copolymerisate in Form eines feinen Granulates. Als Puffer wird eine 0,1 M Ammoniumbikarbonatlösung verwendet. Es ist empfehlenswert, die Elektrophorese bei einer Feldstärke von 4—6 Volt/cm vorzunehmen. Das Protein $PP_{16}$ wandert im elektrischen Feld schneller als die $alpha_1$-Globuline. Die das neue Protein enthaltende Zone wird nach der Auftrennung herausgeschnitten und mit Wasser eluiert. Die Eluate werden anschließend lyophilisiert oder auf einem Ultrafilter eingeengt (Fraktion E).

F) Hochreinigung von $PP_{16}$ mit Immunadsorbentien

$PP_{16}$ in Fraktion E ist noch mit kleinen Mengen von Serumproteinen (hauptsächlich Albumin) und anderen Plazenta-Gewebsproteinen (hauptsächlich humanes Plazenta-Laktogen (hPL) daneben aber auch noch $PP_8$ und $PP_{10}$) verunreinigt. Die Entfernung dieser Begleitproteine gelingt durch inverse oder negative Immunadsorption, d.h. mit Hilfe von trägergebundenen Antikörpern gegen die noch als Verunreinigung vorliegenden Proteine (Fraktion F).

G) Gelfiltration an Sephadex® G-100

$PP_{16}$ ist ein relativ instabiles Protein. Währen der Reinigung werden die Moleküle zum Teil in höhermolekulare Aggregate, zum Teil in niedermolekulare Spaltstücke umgewandelt. Zur Abtrennung dieser Sekundärprodukte wird Fraktion F nochmals an Sephadex G-100 gelfiltriert. Die Hauptfraktion mit Molekulargewicht um 50 000 wird abgetrennt, gegen Wasser dialysiert und lyophilisiert.

**Patentansprüche für die Vertragsstaaten: BE, CH, DE, FR, GB, IT, LI LU, NL, SE**

1. Protein $PP_{16}$, gekennzeichnet durch
a) einen Kohlenhydratanteil von 4,3±1,6%, bestehend aus 3,4±1,2% Hexosen, 0,24±0,07% Hexosaminen, 0,06±0,03% Fucose und 0,6±0,3% Neuraminsäure;
b) einen Sedimentationskoeffizienten $S_{20,w}^0$ von 4,6±0,4 S;
c) ein im Natriumdodecylsulfat (SDS)-haltigen Polyacrylamidgel bestimmtes Molekulargewicht von 46 000±3 000;
d) einen Extinktionskoeffizienten $E_{1\,cm}^{1\%}$ (280 nm) von 8,82±0,5;
e) eine elektrophoretische Beweglichkeit im Bereich des Albumins und
f) einen isoelektrischen Punkt von 4,7±0,2.

2. Verfahren zur Herstellung eines Proteins, dadurch gekennzeichnet, daß eine Flüssigkeit, die das Protein enthält, das
a) einen Kohlenhydratanteil von 4,3±1,6%, bestehend aus 3,4±1,2% Hexosen, 0,24±0,07% Hexosaminen, 0,06±0,03% Fucose und 0,6±0,3% Neuraminsäure;
b) einen Sedimentationskoeffizienten $S_{20,w}^0$ von 4,6±0,4 S;
c) ein im Natriumdodecylsulfat (SDS)-haltigen Polyacrylamidgel bestimmtes Molekulargewicht von 46 000±3 000;
d) einen Extinktionskoeffizienten $E_{1\,cm}^{1\%}$ (280 nm) von 8,82±0,5;
e) eine elektrophoretische Beweglichkeit im Bereich des Albumins und
f) einen isoelektrischen Punkt von 4,7±0,2 hat, einer oder mehreren zur Isolierung von Proteinen bekannten Verfahrensmaßnahmen unterworfen wird, wobei jeweils das Material gewonnen wird, welches das Protein mit den angegebenen Eigenschaften angereichert enthält, und das Protein gewinnt.

3. Verfahren zur Anreicherung des Proteins, das
a) einen Kohlenhydratanteil von 4,3±1,6%, bestehend aus 3,4±1,2% Hexosen, 0,024±0,07% Hexosaminen, 0,06±0,03% Fucose und 0,6±0,3% Neuraminsäure;
b) einen Sedimentationskoeffizienten $S_{20,w}^0$ von 4,6±0,4 S;
c) ein im Natriumdodecylsulfat (SDS)-haltigen Polyacrylamidgel bestimmtes Molekulargewicht von 46 000±3 000;
d) einen Extinktionskoeffizienten $E_{1\,cm}^{1\%}$ (280 nm) von 8,82±0,5;
e) eine elektrophoretische Beweglichkeit im Bereich des Albumins und
f) einen isoelektrischen Punkt von 4,7±0,2 hat, dadurch gekennzeichnet, daß eine Lösung, die dieses Protein enthält, mindestens einer der folgenden Maßnahmen unterworfen wird und die an $PP_{16}$ angereicherte Fraktion gewonnen wird:
a) Fällung mit Ammoniumsulfat im pH-Bereich von 5 bis 8 und bei 30—60% Sättigung;
b) Fällung mit einer wasserlöslichen Acridinbase bei einem pH-Wert zwischen 4 und 9 und einer Konzentration von 0,2—0,8% (w/v);
c) Ausfällung von Begleitproteinen durch Einstellen eines pH-Wertes von 5—6 in einer verdünnten Salzlösung;
d) präparative Zonenelektrophorese bei pH 8 und Gewinnung der Fraktion mit Albuminbeweglichkeit;

e) Gelfiltration zur Gewinnung von Proteinen im Molekulargewichtsbereich von 20 000 bis 70 000;

f) Adsorption an schwach basische Ionenaustauscher und Elution des Proteins.

4. Verwendung des Proteins nach Anspruch 1 zur Gewinung eines Antiserums zum immunologischen Nachweis und zur Bestimmung dieses Proteins.

**Patentansprüche für den Vertragsstaat: AT**

1. Verfahren zur Herstellung eines Proteins, dadurch gekennzeichnet, daß eine Flüssigkeit, die das Protein enthält, das

a) einen Kohlenhydratanteil von 4,3±1,6%, bestehend aus 3,4±1,2% Hexosen, 0,24±0,07% Hexosaminen, 0,06±0,03% Fucose und 0,6±0,3% Neuraminsäure;

b) einen Sedimentationskoeffizienten $S^0_{20,w}$ von 4,6±0,4 S;

c) ein im Natriumdodecylsulfat (SDS)-haltigen Polyacrylamidgel bestimmtes Molekulargewicht von 46 000±3 000;

d) einen Extinktionskoeffizienten $E^{1\%}_{1\,cm}$ (280 nm) von 8,82±0,5;

e) eine elektrophoretische Beweglichkeit im Bereich des Albumins und

f) einen isoelektrischen Punkt von 4,7±0,2 hat, einer oder mehreren zur Isolierung von Proteinen bekannten Verfahrensmaßnahmen unterworfen wird, wobei jeweils das Material gewonnen wird, welches das Protein mit den angegebenen Eigenschaften angereichert enthält, und das Protein gewinnt.

2. Verfahren zur Anreicherung des in Anspruch 1 bezeichneten Proteins, dadurch gekennzeichnet, daß eine Lösung, die dieses Protein enthält, mindestens einer der folgenden Maßnahmen unterworfen wird und die an diesem Protein angereicherte Fraktion gewonnen wird;

a) Fällung mit Ammoniumsulfat im pH-Bereich von 5—8 und bei 30—60% Sättigung;

b) Fällung mit einer wasserlöslichen Acridinbase bei einem pH-Wert zwischen 4 und 9 und einer Konzentration von 0,2—0,8% (w/v);

c) Ausfällung von Begleitproteinen durch Einstellen eine pH-Wertes von 5—6 in einer verdünnten Salzlösung;

d) präparative Zoneelektrophorese bei pH 8 und Gewinnung der Fraktion mit Albuminbeweglichkeit;

e) Gelfiltration zur Gewinnung von Proteinen im Molekulargewichtsbereich von 20 000 bis 70 000;

f) Adsorption an schwach basische Ionenaustauscher und Elution des Proteins.

3. Verwendung des nach einem der Ansprüche 1 und 2 erhältlichen Proteins zur Gewinnung eines Antiserums zum immunologischen Nachweis und zur Bestimmung dieses Proteins.

**Revendications pour les Etats Contractants BE, CH, DE, FR, GB, IT, LI, LU, NL, SE**

1. Protéine PP$_{16}$, caractérisé par:

a) une proportion d'hydrates de carbone de 4,3±1,6%, consistant en 3,4±1,2% d'hexoses, 0,24±0,07% d'hexosamines, 0.06±0,03% de fucose et 0,6±0,3% d'acide neuraminique;

b) un coefficient de sédimentation $S^0_{20,w}$ de 4,6±0,4 S;

c) une masse moléculaire de 46 000±3 000, déterminée dans un gel de polyacrylamide contenant du dodécyl sulfate de sodium (SDS);

d) un coefficient d'extinction $E^{1\%}_{1\,cm}$ (280 nm) de 8,82±0,5;

e) une mobilité électrophorétique dans le domaine de l'albumine et

f) un point isoélectrique de 4,7±0,2.

2. Procédé de préparation d'une protéine, caractérisé en ce qu'on soumet un liquide qui contient la protéine qui a

a) une proportion d'hydrates de carbone de 4,3±1,6%, consistant en 3,4±1,2% d'hexoses, 0,24±0,07% d'hexosamines, 0,06±0,03% de fucose et 0,6±0,3% d'acide neuraminique;

b) un coefficient de sédimentation $S^0_{20,w}$ de 4,6±0,4 S;

c) une masse moléculaire de 46 000±3 000, determinée dans un gel de polyacrylamide contenant du dodécyl sulfate de sodium (SDS);

d) un coefficient d'extinction $E^{1\%}_{1\,cm}$ (280 nm) de 8,82±0,5;

e) une mobilité électrophorétique dans le domaine de l'albumine et

f) un point isoélectrique de 4,7±0,2, à une ou plusieurs mesures opératoires connues pour l'isolement des protéines, par chacune desquelles on produit le matériau qui contient la protéine enrichie, avec les propriétés indiquées, et on obtient la protéine.

3. Procédé d'enrichissement de la protéine qui

a) a une proportion d'hydrates de carbone de 4,3±1,6% consistant en 3,4±1,2% d'hexoses, 0,24±0,07% d'hexosamines, 0,06±0,03% de fucose et 0,6±0,3% d'acide neuraminique;

b) un coefficient de sédimentation $S^0_{20,w}$ de 4,6±0,4 S;

c) une masse moléculaire de 46 000±3 000, déterminée dans une gel de polyacrylamide contenant du dodécyl sulfate de sodium (SDS);

d) un coefficient d'extinction $E^{1\%}_{1\,cm}$ (280 nm) de 8,82±0,5; et

e) une mobilité électrophorétique dans le domaine de l'albumine et

f) un point isoélectrique de 4,7±0,2, caractérisé en ce qu'on soumet une solution qui contient cette protéine, à au moins une des mesures suivantes et on obtient une fraction enriche en PP$_{16}$:

a) précipitation avec du sulfate d'ammonium dans un domaine de pH de 5 à 8 et à 30 à 60% de saturation;

b) précipitation avec une base d'acridine soluble dans l'eau à une valeur de pH comprise entre 4 et 9 et à une concentration de 0,2 à 0,8% (poids/volume);

c) précipitation des protéines secondaires par ajustage à une valeur de pH de 5 à 6 dans une solution de sel diluée;

d) électrphorèse de préparation par zones à un pH de 8 et obtention de la fraction ayant la mobilité de l'albumine;

e) filtration sur gel pour obtenir des protéines ayant une masse moléculaire dans le domaine de 20 000 à 70 000;

f) adsorption sur un échangeur d'ions faiblement basique et élution de la protéine.

4. Utilisation de la protéine selon la revendication 1, pour la production d'un antisérum pour la détection immunologique et la détermination de cette protéine.

**Revendications pour l'Etat Contractant: AT**

1. Procédé de préparation d'une protéine, caractérisé en ce qu'on soumet un liquide qui contient la protéine qui a

a) une proportion d'hydrates de carbone de 4,3±1,6%, consistant en 3,4±1,2% d'hexoses, 0,24±0,07% d'hexosamines, 0,06±0,03% de fucose et 0,6±0,3% d'acide neuramique;

b) un coefficient de sédimentation $S_{20,w}^0$ de 4,6±0,4 S;

c) une masse moléculaire de 46 000±3 000, déterminée dans un gel de polyacrylamide contenant du dodécyl sulfate de sodium (SDS);

d) un coefficient d'extinction $E_{1cm}^{1\%}$ (280 nm) de 8,82±0,5;

e) une mobilité électrophorétique dans le domaine de l'albumine et

f) un point isoélectrique de 4,7±0,2,

à une ou plusieurs mesures opératoires connues pour l'isolement des protéines, par chacune desquelles on produit le matériau qui contient la protéine enrichie avec les propriétés indiquées, et on obtient la protéine.

2. Procédé d'enrichissement de la protéine définie à la revendication 1, caractérisé en ce qu'on soumet une solution qui contient cette protéine, à au moins une des mesures suivantes et on obtient la fraction enrichie en cette protéine:

a) précipitation avec du sulfate d'ammonium dans un domaine de pH de 5 à 8 et à 30 à 60% de saturation;

b) précipitation avec un base d'acridine soluble dans l'eau à une valeur de pH entre 4 et 9 et à une concentration de 0,2 à 0,8% (poids/volume);

c) précipitation des protéines secondaires par ajustage à une valeur de pH de 5 à 6 dans une solution de sel diluée;

d) électrophorèse de préparation par zones à un pH de 8 et obtention de la fraction ayant la mobilité de l'albumine;

e) filtration sur gel pour l'obtention de protéines ayant une masse moléculaire dans le domaine de 20 000 à 70 000;

f) adsorption sur un échangeur d'ions faiblement basique et élution de la protéine.

3. Utilisation de la protéine que l'on peut obtenir selon l'une des revendications 1 et 2 pour la production d'un antisérum pour la détection immunologique et la détermination de cette protéine.

**Claims for the Contracting States: BE, CH, DE, FR, GB, IT, LI, LU, NL, SE**

1. The protein PP$_{16}$, which has

a) carbohydrate content of 4.3±1.6%, comprising 3.4±1.2% of hexoses, 0.24±0.7% of hexosamines, 0.06±0.03% of fucose and 0.6±0.3% of neuraminic acid;

b) a sedimentation coefficient $S_{20,w}$ of 4.6±0,4 S;

c) a molecular weight, determined in polyacrylamide gel containing sodium dodecyl-sulfate (SDS), of 46,000±3,000;

d) an extinction coefficient $E_{1cm}^{1\%}$ (280 nm) of 8,82±0.5;

e) an electrophoretic mobility in the range of albumin and

f) an isoelectric point of 4.7±0.2.

2. A process for the preparation of a protein, which comprises subjecting a liquid containing the protein, which has

a) a carbohydrate content of 4.3±1.6%, comprising 3.4±1.2% of hexoses, 0.24±0.07% of hexosamines, 0.06±0.03% of fucose and 0.6±0.3% of neuraminic acid;

b) a sedimentation coefficient $S_{20,w}$ of 4.6±0.4 S;

c) a molecular weight, determined in polyacrylamide gel containing sodiumdodecyl-sulfate (SDS), of 46,000±3,000;

d) an extinction coefficient $E_{1cm}^{1\%}$ (280 nm) of 8.82±0.5;

e) an electrophoretic mobility in the range of albumin and

f) an isoelectric point of 4.7±0.2,

to one or more process measures known for the isolation of proteins, in each case the material which contains, in concentrated form, the protein with the given properties being isolated, and the protein is isolated.

3. A process for concentrating the protein which has

a) a carbohydrate content of 4.3±1.6%, comprising 3.4±1.2% of hexoses, 0.24±0.07% of hexosamines, 0.06±0.03% of fucose and 0.6±0.3% of neuraminic acid;

b) a sedimentation coefficient $S_{20,w}$ of 4.6±0,4 s;

c) a molecular weight, determined in polyacrylamide gel containing sodium dodecyl-sulfate (SDS), of 46,000±3,000;

d) an extinction coefficient $E_{1cm}^{1\%}$ (280 nm) of 8,82±0.5;

e) an electrophoretic mobility in the range of albumin and

f) an isoelectric point of 4.7±0.2.

which comprises subjecting a solution containing this protein to at least one of the following measures and isolating the PP$_{16}$-enriched fraction:

a) precipitation with ammonium sulfate in the pH range from 5 to 8 and at 30—60% saturation;

b) precipitation with a water

soluble acridine base at a pH value between 4 and 9 and a concentration of 0.2—0.8% (w/v);

c) precipitation of concomitant proteins by adjusting the pH value to 5—6 in a dilute salt solution;

d) preparative zone electrophoresis at pH 8 and isolation of the fraction having the mobility of albumin;

e) gel filtration to obtain proteins in the molecular weight range from 20,000 to 70,000;

f) adsorption onto weakly basic ion exchangers and elution of the protein.

4. Use of the protein as claimed in claim 1 for obtaining an antiserum for immunological detection and for determination of this protein.

## Claims for the Contracting State: AT

1. A process for the preparation of a protein, which comprises subjecting a liquid containing the protein, which has

a) a carbohydrate content of 4.3±1.6%, comprising 3.4±1.2% of hexoses, 0.24±0.07% of hexosamines, 0.06±0.03% of fucose and 0.6±0.3% of neuraminic acid;

b) a sedimentation coefficient $S_{20,w}$ of 4.6±0.4 S;

c) a molecular weight, determined in polyacrylamide gel containing sodiumdodecy-sulfate (SDS), of 46,000±3,000;

d) an extinction coefficient $E_{1\,cm}^{1\%}$ (280 nm) of 8.82±0.5;

e) an electrophoretic mobility in the range of albumin and

f) an isoelectric point of 4.7±0.2, to one or more process measures known for the isolation of proteins, in each case the material which contains, in concentrated form, the protein with the given properties being isolated, and isolating the protein.

2. A process for concentrating the protein according to claim 1 which comprises subjecting a solution containing this protein to at least one of the following measures and isolating the protein-enriched fraction;

a) precipitation with ammonium sulfate in the pH range from 5 to 8 and at 30—60% saturation;

b) precipitation with a water-soluble acridine base at a pH value between 4 and 9 and a concentration of 0.2—0.8% (w/v);

c) precipitation of concomitant proteins by adjusting the pH value to 5—6 in a dilute salt solution;

d) preparative zone electrophoresis at pH 8 and isolation of the fraction having the mobility of albumin;

e) gel filtration to obtain proteins in the molecular weight range from 20,000 to 70,000;

f) absorption onto weakly basic ion exchangers and elution of the protein.

3. Use of the protein obtainable according to claim 1 or 2 for obtaining an antiserum for the immunological detection and for the determination of this protein.

⊖ ⊕

Fig.1a
HS

Anti-HS

Fig.1b
PP₁₆

Anti-PP₁₆